# EUROPÄISCHE PATENTSCHRIFT

(11) **EP 0 102 540 B1**
(45) Veröffentlichungstag und Bekanntmachung des Hinweises auf die Patenterteilung: **02.01.1986**
(21) Anmeldenummer: 83107781.3
(22) Anmeldetag: 08.08.1983
(51) Int. Cl.: C07D 405/06, C07D 413/14, C07D 409/14, C07D 405/14, A01N 43/653, A01N 43/80, C07D 317/26

(54) **Beta-Triazolylketale, Verfahren zu ihrer Herstellung und diese enthaltende Fungizide**
Beta-triazolyl ketals, process for their preparation and fungicides containing them
Bêta-triazolylcétals, leur procédé de préparation et les fongicides les contenant

(30) Priorität: 24.08.1982 DE 3231347
(43) Veröffentlichungstag der Anmeldung: 14.03.1984
(73) Patentinhaber: BASF Aktiengesellschaft, 67063 Ludwigshafen (DE)
(72) Erfinder: Meyer, Norbert, Dr., D-6802 Ladenburg (DE); Renzea, Costin, Dr., D-6900 Heidelberg (DE); Feuerherd, Karl-Heinz, Dr., Ohta-ku Tokyo 145 (JP); Pommer, Ernst-Heinrich, Dr., D-6703 Limburgerhof (DE); Ammermann, Eberhard, Dr., D-6700 Ludwigshafen (DE)

## Beschreibung

Die vorliegende Erfindung betrifft neue Beta-Triazolylketale, Verfahren zu ihrer Herstellung, diese Verbindungen enthaltende fungizide Mittel und ihre Verwendung als Fungizide.

Es ist bekannt, daß Triazolderivate wie beispielsweise 2,2-Dimethyl-4-(1,2,4-triazol-1-yl)-5-phe- nylpentan-3-on (DE-OS 26 38 470) fungizide Wirksamkeit besitzen. Die Wirkung dieser Stoffe ist bei niedrigen Aufwandmengen und Konzentrationen nicht immer ausreichend. Darüber hinaus ist ihre fungitoxische Wirkung mit einer hohen Phytotoxität verbunden, so daß bei den für die Bekämpfung von phytopathogenen Pilzen, beispielsweise bei der Bekämpfung von Mehltau, Schorf oder Rostpilzen notwendigen Konzentrationen auch wichtige Kulturpflanzen geschädigt oder in ihrem Wachstum stark gehemmt werden. Aus diesen Gründen sind die zur Bekämpfung von phytopathogenen Pilzen nur schlecht geeignet.

Es wurde nun gefunden, daß Verbindungen der Formel I 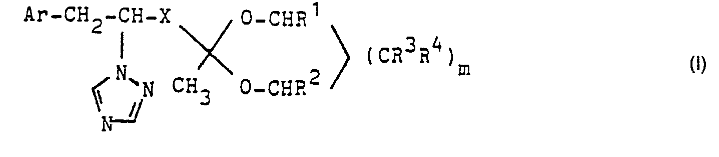 in der
_{R}¹, R², R³, R⁴ verschieden oder gleich sind und Wasserstoff oder Alkyl mit 1 bis 5 C-Atomen bedeuten,

Ar Furanyl, Thienyl, Biphenylyl, Isoxazolyl, Naphthyl oder Phenyl bedeutet, wobei der Phenylrest durch Fluor, Chlor, Brom, lod, Nitro, Trifluormethyl, Cyano weiterhin durch Alkyl, Alkoxy oder Alkenyl mit jeweils 1 oder 2 bis 5 C-Atomen und ferner durch Phenoxy substituiert sein kann, der Isoxazolylrest durch Alkyl mit 1 bis 5 C-Atomen substituiert sein kann und

X eine -CO-, -CH(OH)-, -CR⁵(OH)- oder -CH(OR⁶)-Gruppe bedeutet, wobei R⁵ einen Alkylrest mit 1 bis 4 C-Atomen oder einen Alkenylrest mit 2 bis 4 C-Atomen oder einen Benzylrest, der durch Halogen substituiert sein kann, bedeutet,

R⁶ einen Alkyrest mit 1 bis 8 C-Atomen oder einen gegebenenfalls durch Chlor substituierten Alkenylrest mit 2 bis 5 C-Atomen oder einen Alkinylrest mit 3 oder 4 C-Atomen oder Benzyl bedeutet, wobei der Benzylrest durch Fluor, Chlor, Brom, Nitro, Cyano, Trifluormethyl, ferner durch Alkyl oder Alkoxy mit 1 bis 4 C-Atomen substituiert sein kann, oder R⁶ einen --CO-R7-Rest bedeutet, wobei R⁷ einen gegebenenfalls durch Halogen, C₁-C₄-Alkoxy, Oxo (= 0) oder Carboxy-C₁-C₄-alkyl substituierten Alkylrest mit 1 bis 5 C-Atomen oder einen aromatischen Rest bedeutet und

m eine ganze Zahl von 0 bis 3 ist sowie deren Salze und Metallkomplexverbindungen ausgezeichnet wirksam gegen Schadpilze sind und sehr gute Pflanzenverträglichkeit zeigen.

Die neuen Verbindungen der Formel I enthalten chirale Zentren und werden im allgemeinen in Form von Racematen oder als Diastereomerengemische von erythro- sowie threo-Formen erhalten. Die erythro-und threo-Diastereomeren lassen sich bei einigen der neuen Verbindungen beispielsweise durch Löslichkeitsunterschiede oder durch Säulenchromatographie trennen und in reiner Form isolieren. Aus solchen einheitlichen Diastereomerenpaaren kann man mit bekannten Methoden einheitliche Enantiomere erhalten. Diese und die oben genannten Gemische werden von der vorliegenden Erfindung umfaßt. Für die Anwendung der neuen Verbindungen als Fungizide kann man sowohl die einheitlichen Diastereomeren bzw. Enantiomeren wie auch deren bei der Synthese anfallenden Gemische verwenden. Bevorzugt werden die letzteren verwendet.

_{R}^{l}, _{R}², _{R}³, R⁴ bedeuten beispielsweise Wasserstoff, Methyl, Ethyl, n-Propyl, Isopropyl, n-Butyl, sek.-Butyl, iso-Butyl, tert.-Butyl, n-Pentyl und Neopentyl.

Ar bedeutet beispielsweise 2-Furanyl, 2- und 3-Thienyl, 3-und 5-lsoxazolyl, 3-Methyl-5-isoxazolyl, 3-tert.-Butyi-5-isoxazoiyi, 4-Biphenylyl, 1-und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-methoxyphenyl, 2,3,4-Trichlorphenyl, 2-Methoxyphenyl, 2,4-Dimethoxyphenyl, 3-Methoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 3-Nitrophenyl, 4-Nitrophenyl, 3-Trifluormethylphenyl, 4-Trifluormethylphenyl, 4-Phenoxyphenyl und 4-Cyanophenyl.

X bedeutet beispielsweise eine Carbonylgruppe (C = 0) oder die entsprechenden Alkohole (-CHOH) sowie die entsprechenden Ether (-CH-OR⁶) oder die entsprechenden Ester (―CH―OCOR⁷). R⁶ bedeutet beispielsweise Methyl, Ethyl n-Propyl, n-Butyl, 4-Chlorbutyl, n-Pentyl, n-Hexyl, Allyl, Buten-2-yl, Propargyl, Benzyl, 4-Fluorbenzyl, 4-Chlorbenzyl, 4-Brombenzyl, 2,4-Dichlorbenzyl und 4-Trifluormethylbenzyl. R⁷ bedeutet vorzugsweise Methyl, Ethyl, n-Propyl, Isopropyl, Chlormethyl, Chlorpropyl, Methoxymethyl, Vinyl und Propenyl. X bedeutet ferner CR⁵0H, wobei R⁵ beispielsweise Methyl, Ethyl, Propyl, Butyl, Vinyl, Propenyl, Butenyl, Benzyl, 4-Chlorbenzyl bedeutet.

Säureadditionssalze sind z. B. die Salze mit Salzsäure, Schwefelsäure.

Metallkomplexe sind. z. B. die Komplexe mit Kupfer, Zink.

Die neuen Verbindungen lassen sich herstellen, indem man
a) 1,2,4-Triazol mit alpha-Bromketonen der Formel II 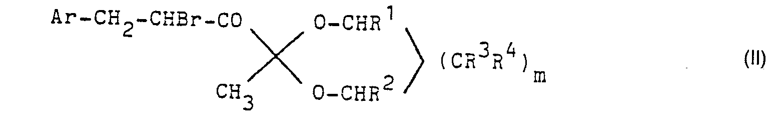 worin R¹ bis R⁴, Ar und m die oben angegebene Bedeutung haben oder
b) ein Arylmethylhalogenid der Formel III 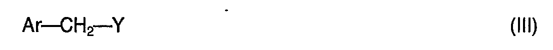 worin Ar die oben genannte Bedeutung hat und Y Chlor oder Brom ist, mit einem β-Triazolylketal der Formel IV 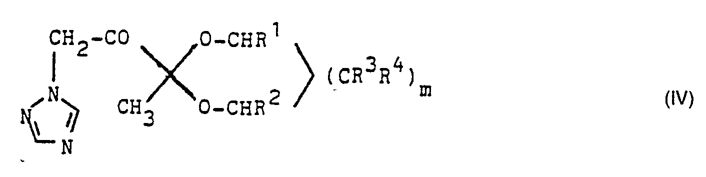 worin R¹ bis R⁴ und m die oben genannte Bedeutung haben, umsetzt, und die so erhaltenen Verbindungen gegebenenfalls anschließend mit Grignard-Reagenzien umsetzt oder reduziert und- falls gewünscht - danach verethert oder verestert und gegebenenfalls eine Säure oder ein Metallsalz addiert.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethyl-ether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische. Geeignete Basen, die gegebenenfalls auch als säurebindende Mittel bei der Reaktion verwendet werden können, sind beispielsweise Alkalihydroxide wie Lithium, Natrium- oder Kaliumhydroxid ; Alkalicarbonate wie Natrium- oder Kaliumhydroxid ; Alkalicarbonate wie Natrium- oder Kaliumcarbonat oder Natrium- und Kaliumhydrogencarbonat, Überschüsse an 1,2,4-Triazol, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder-iodid, Benzyltriethylammonium-chlorid oder- bromid oder Kronenether wie 12-Krone-4, 15-Krone-5, 18-Krone-6, Dibenzo-18-Krone-6 oder Dicyclohexano-18-Krone-6 in Frage.

Die Umsetzungen werden im algemeinen bei Temperaturen zwischen 20 und 150 °C, drucklos oder unter Druck, kontinuierlich oder diskontinuierlich durchgeführt.

Die Bromketone der Formel II können beispielsweise durch Bromierung von Verbindungen der Formel V 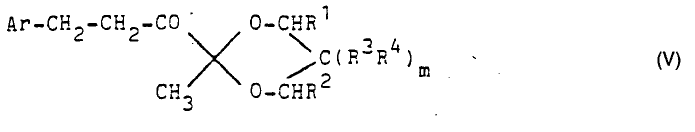 in welcher Ar, R¹ bis R⁴ und m die oben angegebenen Bedeutungen haben, mit Brom in Formamid nach H. Bredereck und Mitarb., Chem. Ber. 93, 2 083 (1960) oder mit Dioxan-Dibromid nach S. J. Pasaribu und L.R. Williams, Aust. J. Chem. 26, 1 327 (1973) oder mit dem Komplex (Pyrroildon)₃ · HBr - Br₂ nach D. _{C}. Awang und Mitarb., Can. J. Chem. 47, 706 (1969) erhalten werden.

Die Reaktion b) erfolgt gegebenenfalls in Gegenwart eines Lösungs- oder Verdünnungsmittels und gegebenenfalls unter Zusatz einer starken anorganischen oder organischen Base bei Temperaturen zwischen - 10 und 120 °C. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsufloxid und schließlich Sulfolan.

Geeignete Basen, die gegebenenfalls auch als säurebindende Mittel bei der Reaktion verwendet werden können, sind beispielsweise Alkalihydride wie Lithium, Natrium- und Kaliumhydrid, Alkaliamide wie Natrium- und Kaliumanid, ferner Natrium- oder Kaloium-tert.-butoxid, Lithium-, Natrium- oder Kalium-Triphenylmethyl und Naphthalin-Lithium, -Natrium oder Kalium.

Die Beta-Triazolylketale der Formel IV können beispielsweise durch Umsaetzung von Beta-Halogenketalen der Formel VI 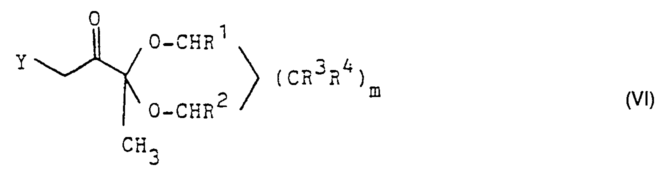 in welcher R¹ bis R⁴ und m die oben angegebenen Bedeutungen haben und Y Chlor oder Brom sein kann, mit 1,2,4-Triazol oder dessen Alkalisalz in einem geeigneten Lösungsmittel erhalten werden.

Beta-Halogenketale der Formel VI erhält man z. B. durch Bromierung von z. T. bekannten Ketalen der Formel VII 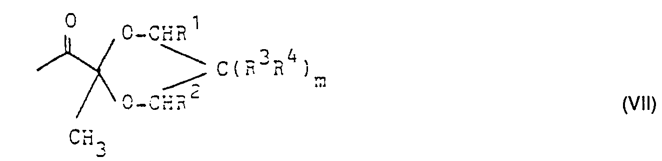 in welcher R¹ bis R⁴ und m die oben angegebenen Bedeutungen haben, beispielsweise mit dem Pyrrolidon-Brom-Komplex nach D. P. Wyman u. P. R. Kaufman, J. Org. Chem. 29, 1956 (1964).

Die Monoketale von Diacetyl werden nach Literaturangaben hergestellt, z. B. C. Henriquez, Rec. Trav. Chim. 54, 737 (1935) oder soweit noch unbekannt, werden sie nach entsprechenden Verfahren erhalten.

Die gemäß a) oder b) erhaltenen Ketone lassen sich gegebenenfalls in Gegenwart eines Lösungs-oder Verdünnungsmitels, bei Temperaturen zwischen - 20 bis 150 °C, bei Normaldruck oder unter erhöhtem Druck mit Wasserstoff in Gegenwart eines Katalysators, mit komplexen Bor- bzw. Aluminiumhydriden, mit Aluminiumisopropoxid und Isopropanol, mit Natriumdithionit oder elektrochemisch reduzieren.

Geeignete Lösungs- oder Verdünngsmittel hierfür sind beispielsweise Wasser, Methanol, Ethanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Palladiumkatalysatoren auf inerten Trägern und hydriert bei Drucken von 2 bis 80 bar bis keine Wasserstoffaufnahme mehr erfolgt.

Für die als Reduktionsmittel verwendbaren Hydride seien beispielsweise Natriumborhydrid oder Lithiumaluminiumhydrid genannt.

Die so erhaltenen Alkohole der Formel I (X = CHOH) lassen sich mit Alkylierungsmitteln der Formel VIII 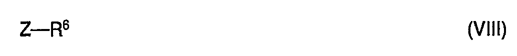 worin R⁶ die oben angegebene Bedeutung hat und Z Chlor oder Brom darstellt, gegebenenfalls in Gegenwart eines Lösungs- oder Verdünnungsmittels die Ein- oder Zweiphasensysteme bilden, gegebenenfalls unter Zusatz einer anorganischen Base und gegebenenfalls unter Zusatz eines Reaktionsbeschleunigers und/oder Phasentransferkatalysators verethern.

Hierfür eignen sich folgende Lösungs- oder Verdünnungsmittel : Diethylether, Tetrahydrofuran, Dixoan, n-Pentan, monohalogenierte Kohlenwasserstoffe mit 2 bis 6 C-Atomen wie beispielsweise Chlorethan, Bromethan, 1-Chlorpropan, 1-Brompropan, 1-Chlorbutan, 1-Brombutan, 1-Chlorpentan, 1-Brompentan, 1-Chlorhexan, 1-Bromhexan, ferner Cyclohexan, Methylenchlorid, Chloroform, Toluol oder Dimethylformamid.

Als anorganische Basen seien beispielsweise genannt : Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magnesiummethylat, Natriumisopropylat oder Kalium-tert.-butylat.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylaminopyridin und 4-Pyrrolidinopyridin, Kronenether wie 12-Krone-4, 14-Krone-5, 18-Krone-6, Dibenzo-18-krone-6 oder Dicyclohexano-18-Krone-6 oder Azole wie Imidazol und 1,2,4-Triazol.

Als Phasentransferkatalysatoren kommen vorzugsweise quaternäre Ammoniumsalze wie Tetrabutylammoniumchlorid, -bromid, -iodid oder -hydrogensulfat, Benzyl-triethyl-ammoniumchlorid, Methyltrioctylammoniumchlorid und -bromid und Phosphoniumsalze wie Tetrabutylphosphoiniumbromid und - iodid und Tetra-n-pentylphosphoniumbromid und- iodid in Frage.

Die Veresterung der Alkohole der Formel (X = CHOH) kann mit Säurechloriden oder -anhydriden der Formeln Y-CO-R⁷ (VIII) oder (R⁷-CO)₂O (IX) gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers erfolgen. Hierfür eignen sich dieselben Lösungsmittel und Basen wie für die Veretherung. Zusätzlich können tertiäre Amine wie Trimethylamin, Triethylamin, N,N-Dimethylanilin, N,N-Dimethylcyclohexylamin, N-Methylpiperidin oder Pyridin als Base verwendet werden.

Mit geeigneten Basen wie z. B. Alkalihydrid wie Natriumhydrid oder Alkali- oder Erdalkalialkoholaten wie Natriummethylat können die Alkohole auch in einer ersten Umsetzung zunächst in ihre Alkoholat-Salze überführt werden und dann als solche zur Reaktion gebracht werden.

Die Ketone I(X = C = 0) lassen sich durch Umsetzung mit Grignard-Reagenzien der Formel IX 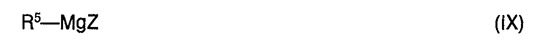 in der R⁵ die oben erläuterte Bedeutung hat und Z Halogen wie lod, Brom oder Chlor sein kann, zu tertiären Alkoholen umwandeln. Die Reaktion findet in Gegenwart inerter Lösungsmittel bei Temperaturen zwischen 0 und 80 °C statt. Prinzipiell sind derartige Reaktionen umfassend beschrieben, vgl. z. B. K. Nützel in Houben-Weyl, Müller, Methoden der organischen Chemie, Georg Thieme Verlag, Stuttgart 1973, Bd. XIII, 2a, S. 53ff.

Die so erhaltenen Verbindungen der Formel I werden nach üblichen Methoden isoliert, gegebenenfalls gereinigt und gegebenefals mit Säuren, z. B. Salzsäure, oder Metallsalzen, z. B. Kupfersulfat, zu Salzen bzw. zu Metall-Komplexen umgesetzt.

### Beispiel 1

### a) Herstellung des Ausgangsmaterials

Zu einer Lösung von 134,0 g (1,03 Mol) 2-Acetyl-2-methyl-1,3-dioxolan und 82,4 g (0,97 Mol) Pyrrolidon in 1 200 ml Tetrahydrofuran wird in 2 Stunden bei 50 °C die Lösung von 479,8 g (0,97 Mol) Pyrrolidon-Brom-Komplex in 1 Liter Tetrahydrofuran zugetropft. Nach achtstündigem Nachrühren bei 50 °C wird der weiße Niederschlag von Pyrrolidon-Hydrobromid abgesaugt, mit 50 ml Tetrahydrofuran gewaschen und as Filtrat im Vakuum eingeengt. Man erhält 200 g rohes, öliges 1-(2-Methyl-1,3-dioxolan-2-yl)-2-brom-ethan-1-on.

Man mischt unter Stickstoff 57,5 g (1,06 Mol) Natriummethylat, 580 ml trockenes Toluol und 66,8 g (0,97 Mol) 1,2,4-Triazol und erhitzt unter kräftigem Rühren und gleichzeitigem Abdestillieren eines Toluol/Methanol-Gemisches bis die Kopftemperatur 110 °C erreicht ist. Nach Abkühlen bis auf 60 _{°}C tropft man dann 220,0 g 1-(2-Methyl-1,3-dioxolan-2-yl)-2-bromethan-1-on zu und rührt noch 2 Stunden bei dieser Temperatur und 12 Stunden bei Raumtemperatur nach. Der Feststoff wird abgesaugt, mit Toluol nachgewaschen und das Filtrat eingeengt und destilliert. Man erhält 73,1 g 1-(2-Methyl-1,3-dioxolan-2-yl)-2-(1,2,4-triazotyt)-1))-ethan-1-on mit Siedepunkt 147°C/0,2 mbar.

IR (Film) : 2975, 1 745, 1 513, 1 273, 1 139, 1 121, 1 036, 1 022, 888, 865 cm-¹

### b) Herstellung des Endproduktes

Zu einer unter reinem Stickstoff gerührten Suspension von 2,9 g (0,092 Mol) Natriumhydrid (80 %ig) in 30 ml trockenem Dimethylformamid wurde die Lösung von 16,5 g (0,084 Mol) 1-(2-Methyl-1,3-dioxolan-2-yl)-2-(1,2,4-traizolyl-(1)-ethan-1-on in 15 ml Dimethylformamid bei 5 bis 10 °C zugetropft. Nach dreistündigem Nachrühren wurde bei 25 °C die Lösung von 16,4 g (0,084 Mol) 2,4-Dichlorbenzylchlorid in 15 ml Dimethylformamid zugetropft und das Reaktionsgemisch weitere 14 Stunden nachgerührt. 100 ml Eiswasser wurden vorsichtig zugetropft und es wurde mit Methylenchlorid extrahiert. Nach Trocknen über Na₂S0₄ wurde die eingeengte organische Phase mit wenig Methyl-tert.-butylether verrieben, abgesaugt und getrocknet. Man erhielt 14,2 g 1-(2-Methyl-1,3-dioxolan-2-yl)-2-(1,2,4-triazolyl-(1))-3-(2,4-dichlorphenyl)-propan-1-on mit Schmelzpunkt 162 bis 165 °C (Verbindung Nr. 1).

### Beispiel 2

Zu einer unter reinem Stickstoff gerührten Suspension von 0,98 ^{g} (0,033 Mol) Natriumhydrid (_{80 %}ig) in 1₃ ml trockenem Dimethylformamid wurden 6,0 g (0,028 Mol) 1-(2,4-Dimethyl-1,3-dioxolan-2-yl)-2-(1,2,4-triazolyl)-(1)-ethan-1-on gelöst in 8 ml Dimethylformamid bei 5 bis 10 °C zugetropft. Man rührte 3 Stunden bei 25 °C nach und tropft dann eine Lösung von 5,55 g (0,028 Mol) 2,4-Dichlorbenzylchlorid in 8 ml Dimethylformamid zu. Es wurde 14 Stunden nachgerührt, vorsichtig mit Wasser hydrolysiert und mit Methylenchlorid extrahiert. Die vereinigten organischen Phasen wusch man dreimal mit 10%iger Salzsäure und anschließend mit verdünnter Natronlauge. Nach Trocknen und Einengen der Lösung erhält man 9,1 g 1-(2,4-Dimethyl-1,3-dioxolan-2-yl)-2-(1,2,4-triazolyl-(1))-3-(2,4-dichlorphenyl)-propan-1- on als zähes Öl (Verbindung Nr. 2).

IR (Film) : 2 995, 2 940, 2 895, 1 744, 1 502, 1 474, 1 276, 1 202, 1 139, 1 103, 1 083, 1 051, 1 030

### Beispiel 3

Zu einer Lösung von 7,0 g (0,0197 Mol) 1-(2-Methyl-1,3-dioxolan-2-yl)-2-(1,2,4-triazolyl)-(1))-3-2,4-dichlorphenyl)-propan-1-on in 40 ml Methanol wurden zwischen 0 und + 5°C 0,41 g (0,01 Mol) Natriumbrohydrid portionsweise zugegeben. Man rührte anschließend 30 Minuten bei 25 °C und 5 Stunden unter Rückfluß. Dann wurde die Lösung eingeengt, der Rückstand in Methylenchlorid gelöst, dreimal mit Wasser gewaschen, über Na₂S0₄ getrocknet und eingeengt. Dabei erhielt man 5,5 g 1-(2,4-Dimethyl-1,3-dioxolan-2-yl)-2-(1,2,4-triazolyl-(1))-3-2,4-dichlorphenyl-propan-1-ol mit Schmp. 108 bis 112°C (Diastereomerengemisch) Verbindung Nr. 3).

Entsprechend wurden diejenigen in der folgenden Tabele angegebenen Verbindungen der Formel I hergestellt, deren Schmelzpunkte (Fp) oder IR-Spektren angegeben sind. Ihre Struktur wurde durch Ultrarot- und Kernresonanz-Spektroskopie sowie durch Elementaranlyse gesichert. Die Verbindungen, bei dene keine Angaben zur physikalischen Chemie gemacht sind, können auf die gleiche Art und Weise wie bei den tatsächlich hergestellten Verbindungen erhalten werden ; es kann erwartet werden, daß sie aufgrund ihrer ähnlichen Konstitution ähnliche Wirkungen wie die näher untersuchten Verbindungen haben.

### (Siehe Tabelle Seite 7 f.)

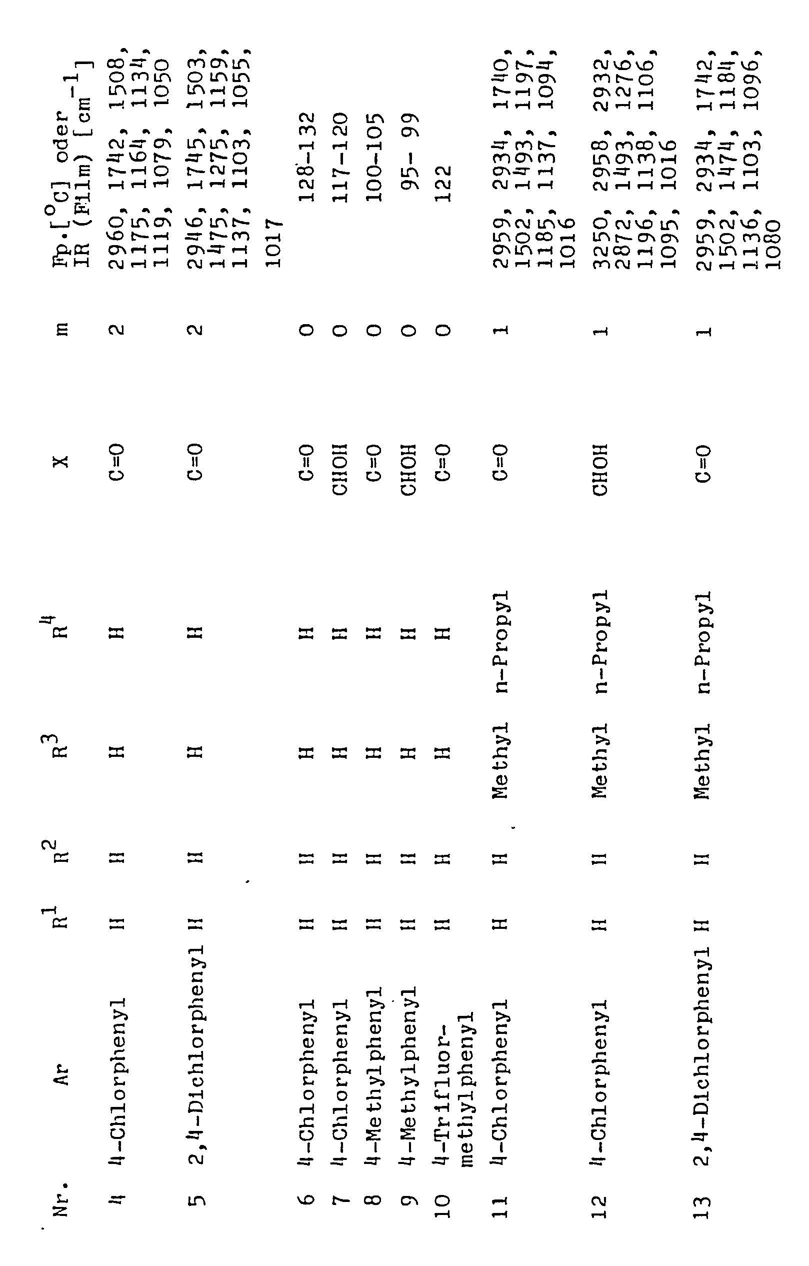

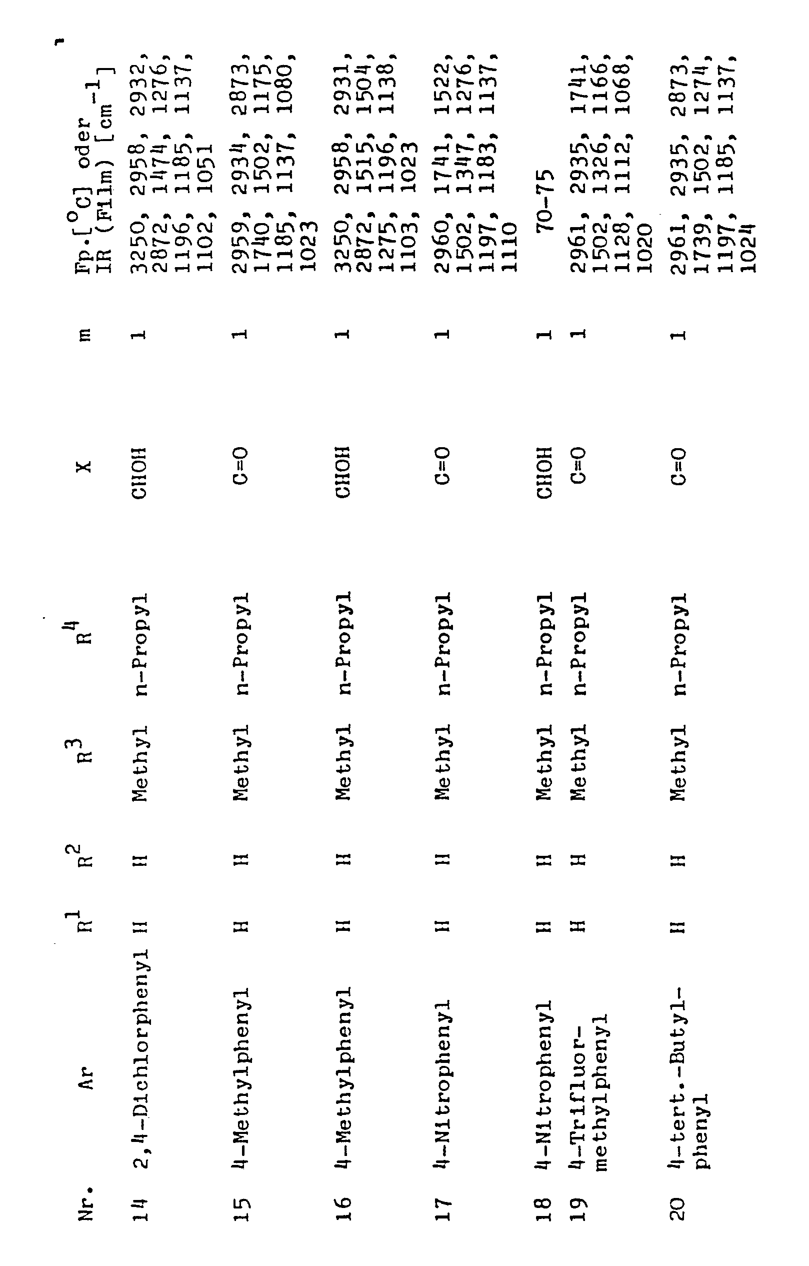

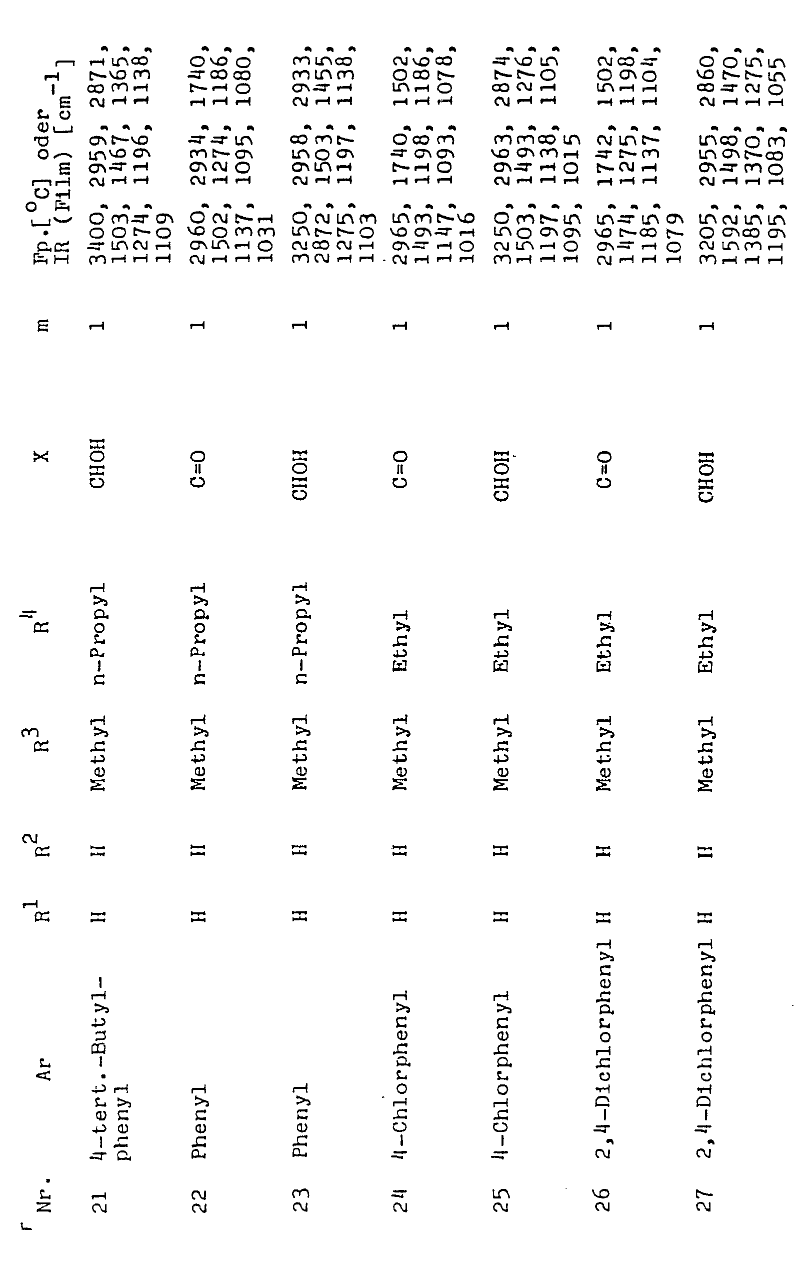

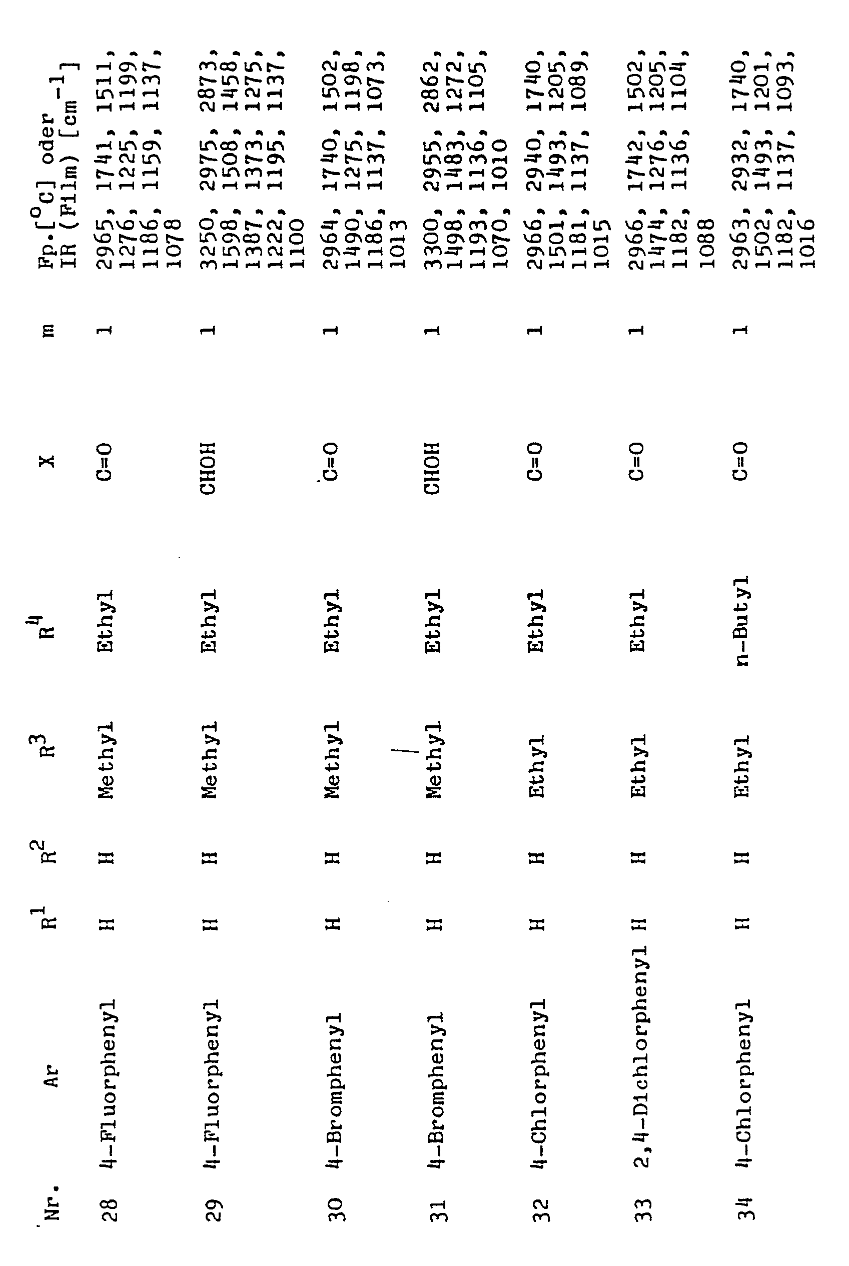

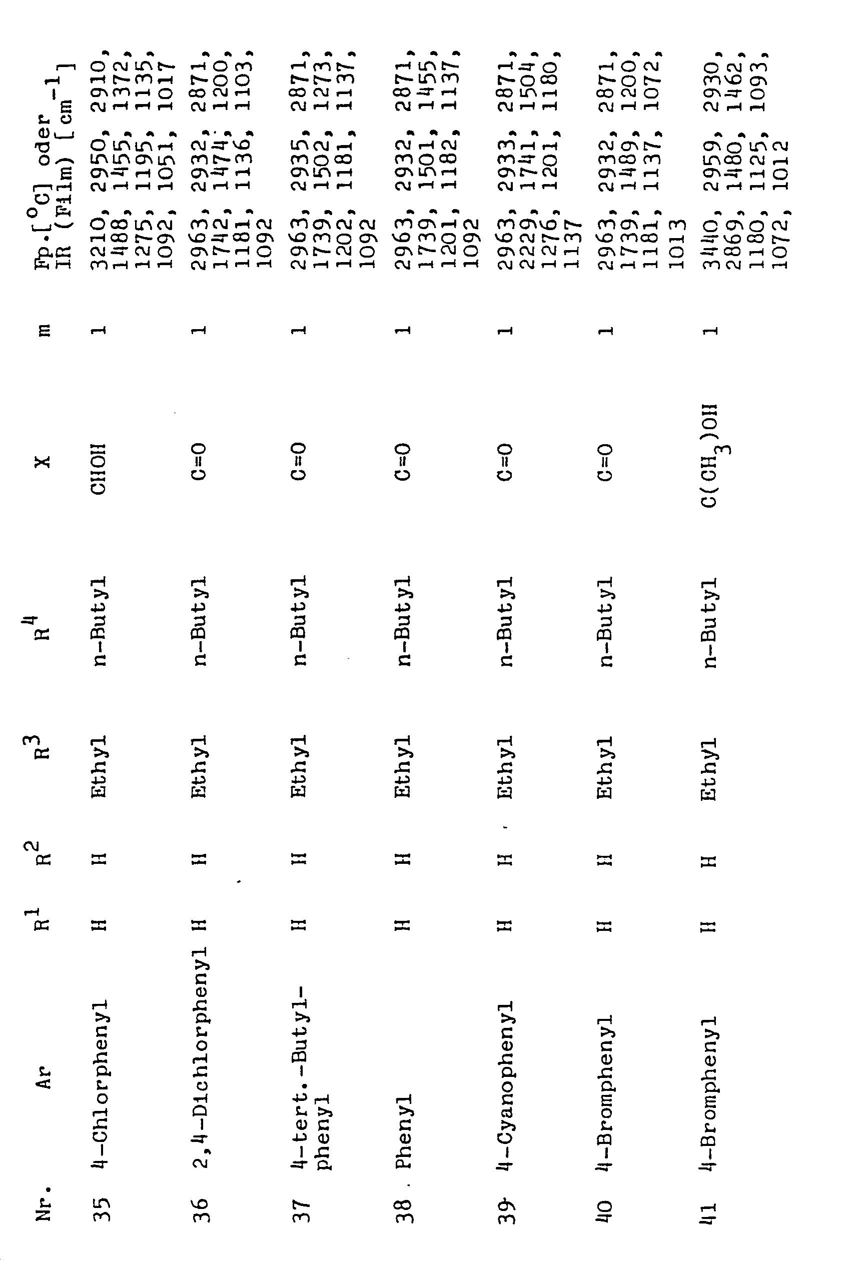

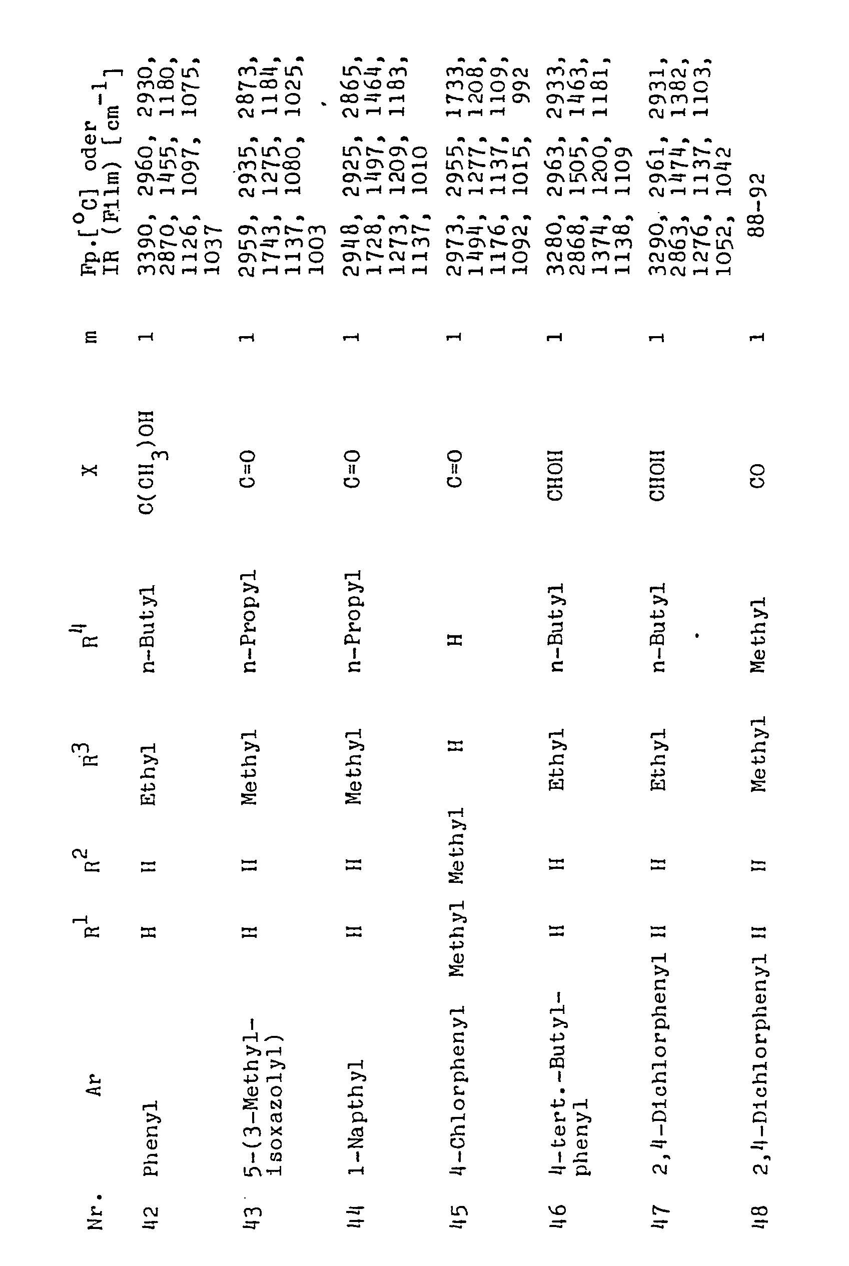

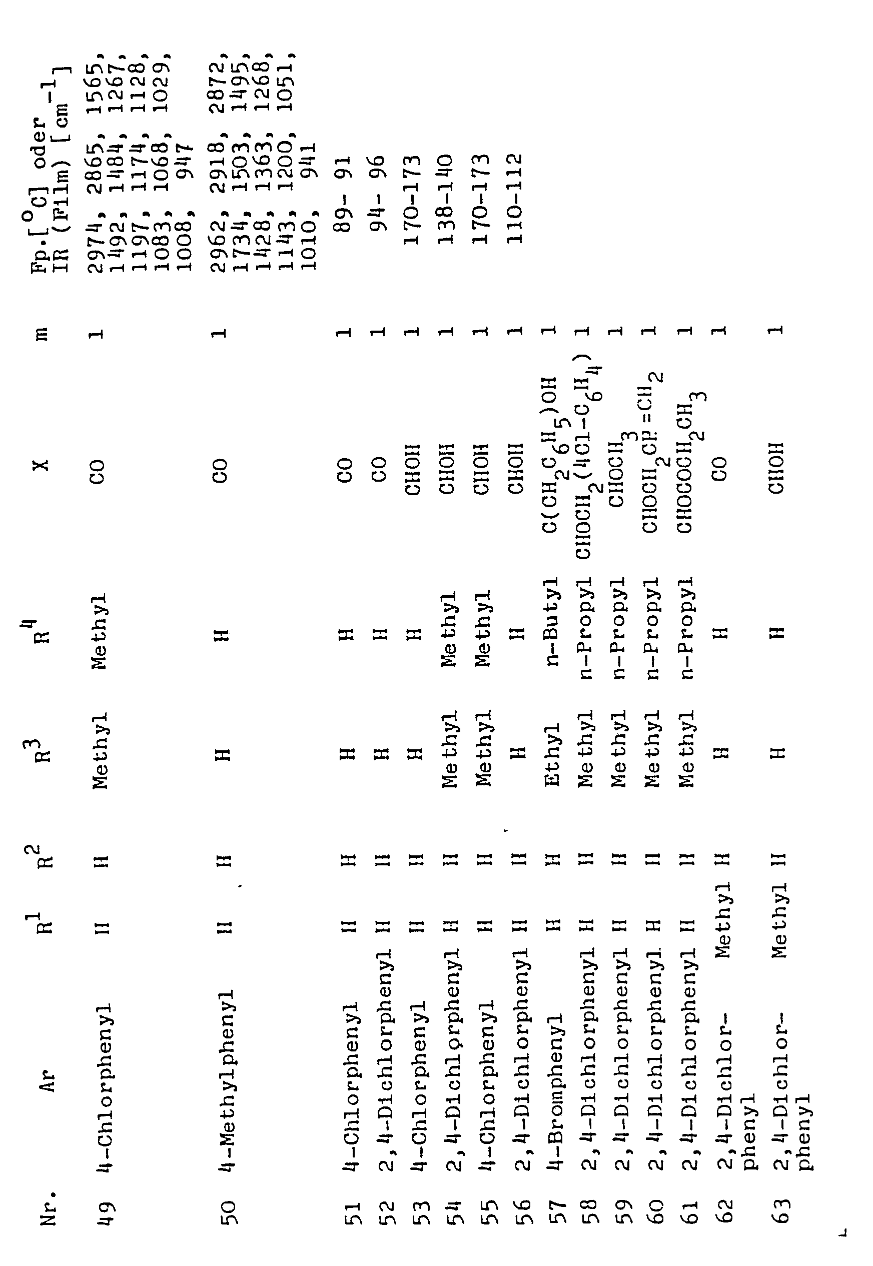

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. zur Bekämpfung holzzerstörender Pilze, wie Coniophora puteana und Polystictus versicolor, verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüsewie Gurken, Bohnen und Kürbisgewächse-.

Die neuen Verbindungen sind insbesondere geeignet zur Bekämpfung folgender Pflanzenkrankheiten :
Erysiphe graminis (echter Mehltau) in Getreide,
Erysiche cichoracearum an Kürbisgewächsen,
Podosphaera leucotricha an Äpfeln,
Uncinula necator an Reben,
Puccinia-Arten an Getreide,
Rhizoctonia solani an Baumwolle
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Phthophthora infestans (falscher Mehltau) an Kartoffeln,
Tomaten,
Pyricularia oryzae an Reis.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Verbindungen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser ; Trägerstoffe wie natürliche Gesteinsmehle, z. B. Kaoline, Tonerden, Talkum, Kreide und synthetische Gesteinsmehle (z. B. hochdisperse Kieselsäure, Silikate) ; Emulgiermittel, wie nichtionogene und anionische Emulgatoren (z. B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha.

Die neuen Verbindungen können auch im Materialschutz u.a. zur Bekämfung holzzerstörender Pilze, wie Coniophora puteana und Polystictus versicolor eingesetzt werden. Bei der Anwendung der Wirkstoffe im Materialschutz, z. B. als Fungizide für Anstrichfarben und Weich-Polyvinylchlorid, betragen die Aufwandmengen 0,05 bis 5 Gew.% Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

I. 20 Gewichtsteile der Verbindung des Beispiels 7 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.
11. 3 Gewichtsteile der Verbindung des Beispiels 29 werden mit 97 Gewichtsteilen feinteiligem Kaolin innig vermischt. Man erhält auf diese Weise ein Stäubemittel, das 3 Gew.% des Wirkstoffs enthält.
111. 30 Gewichtsteile der Verbindung des Beispiels 45 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
IV. 40 Gewichtsteile der Verbindung des Beispiels 49 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.
V. 20 Teile der Verbindung des Beispiels 56 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.
Vl. Man versmischt 90 Gewichtsteile der Verbindung des Beispiels 7 mit 10 Gewichtsteilen N-Methylalpha-pyrrolidon und erhält eine Lösung, die zur Anwendung in Form kleinster Tropfen geeignet ist.
VII. 20 Gewichtsteile der Verbindung des Beispiels 29 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.
VIII. 20 Gewichtsteile der Verbindung des Beispiels 45 werden in einer Mischung gelöst, die aus 4₀ Gewichtsteilen Cyclohexanon, 30 Gewichtsteilen Isobutanol, 20 Gewichtsteilen des Anlagerungsproduktes von 7 Mol Ethylenoxid an 1 Mol lsooctylphenol und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IX. 10 Gewichtsteile der Verbindung des Beispiels 49, 20 Gewichtsteile Polyoxyethylensorbitan- ₘₒₙₒₗₐᵤrat 20 Gewichtsteile Methanol und 50 Gew.-Teile Wasser werden zu einer Lösung verrührt, die _{10 G}ew.% des Wirkstoffs enthält. Durch Hinzufügen von weiterem Wasser können stärker verdünnte Lösungen hergestellt werden.

Die neuen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des Wirkungsspektrums. Bei einer Anzahl solcher Fungizidmischungen treten auch synergistische Effekte auf, d. h. die Wirksamkeit des Kombinatiönsproduktes ist größer als die addierten Wirksamkeiten der Einzelkomponenten.

Fungizide, die mit den neuen Verbindungen kombiniert werden können, sind beispielsweise : Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N'-ethylen-bis-dithiocarbamat)
Ammoniak-Komplex von Zink-(N,N'-propylen-bis-(thiocarbamoyl),
Zink-(N,N'-propylen-bis-(thiocarbamoyl)-disulfid.
Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;
5-Nitro-isophthalsäure-di-isopropylester,
Heterocyclische Strukturen, wie
2-Heptadecyl-2-imidazolin-acetat,
2,4-Dichlor-6-(o-chcloranilino)-s-triazin,
0,0-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-4,5-b)-chinoxalin,
1-(Butylcarbamoyl)-2-benzimidazol-carbaminsäuremethylester,
2-Methoxycarbonylamino-benzimidazol,
2-(Furyl-(2)-benzimidazol,
2-(Th iazo lyl-(4))-benzi m idazo I,
N-(1,1,2,2-Tetrachlorethylthio)-tetrahydrophthalimid,
N-Trichlormethylthio-tetrahydrophthalimid,
N-Dichlorfluormethylthio-N,N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4-H-pyran-3-carbonsäureanilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-lod-benzoesäure-anilid,
N-Formyl-N-morpholin-2,2,2-trichlorethylacetal,
Piperazin-1,4-diylbis-(1-(2,2,2,-trichlor-ethyl)-formamid,
1-(3,4-Dichloranilino)-1-formylamino-2,2,2-trichlorethan,
2,6-Dimethyl-N-tridecyl-morpholin bzw. dessen Salze
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1 H-1,2,4-triazol,
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol, sowie verschiedene Fungizide, wie
Dodecylguanidinacetat,
3-(3-(3,5-Dimethyl-2-oxycyclohexyl)-2-hydroxyethyl)-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethyl-ester,
5-Methyl-5-vinyl-3-(3,5-dichlorphenyl)-2,4-dioxo-1,3-oxazolidin,
3-(3,5-Dichlorphenyl)-5-methyl-5-methoxymethyl-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid.

Die folgenden Versuche erläutern die fungizide Wirkung der neuen Verbindungen. Für diese Versuche wurde als bekannter Vergleichswirkstoff A die Verbindung 2,2-Dimethyl-4-(1,2,4-triazol-1-yl)-5-phenylpentan-3-on (DE-OS 26 38 470) verwendet.

### Versuch 1

### Wirksamkeit gegen Weizenmehltau

Blätter von in Töpfen gewachsenen Weizenkeimlingen der Sorte « Jubilar werden mit wäßriger Spritzbrühe, die 80 % Wirkstoff und 20 % Emulgiermittel in der Trockensubstanz enthält, besprüht undf 24 Stunden nach dem Antrocknen des Spritzbelages mit Oidien (Sporen) des Weizenmehltaus (Erysiphe graminis va. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß die Verbindungen 7, 29, 45, 49 und 56 bei Anwendung als 0,025-, 0,006- oder 0,0015 %ige (Gew.%) Spritzbrühe eine bessere fungizide Wirkung (90 %) zeigten als der Wirkstoff A (80 %).

### Versuch 2

### Wirksamkeit gegen Gurkenmehltau

Blätter von in Töpfen gewachsenen Gurkenkeimligen der Sorte « Chinesische Schlange werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22 °C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß die Verbindungen 2,3,10,12,14,16,19, 24, 25, 26, 27, 29, 31, 32, 33, 34, 35, 40, 41, 42, 43, 44, 45, 46, 47, 54, 55 und 56 bei der Anwendung als 0,025 %ige Spritzbrühe eine bessere fungizide Wirkung (97 %) zeigten als der Wirkstoff A (90 %).

### Versuch 3

### Wirksamkeit gegen Weizenbraunrost

Blätter von in Töpfen gewachsenen Weizensämlingen der Sorte « Jubilar werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22 °C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß die Verbindungen 4, 11, 12, 29, 34 und 55 bei Anwendung als 0,025 %ige Spritzbrühe eine bessere fungizide Wirkung (97 %) zeigten als der Wirkstoff A (90 %).

### Versuch 4

### Wirksamkeit gegen Botrytis cinerea an Paprika

Paprikasämlinge der Sorte « Neusiedler Ideal Elite werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegeden Teil der Blätter bedecken.

Das Ergebnis des Versuches zeigt, daß die Verbindungen 3, 40, 45 und 56 bei Anwendung als 0,05 %ige Spritzbrühe eine bessere fungizide Wirkung (90 %) zeigten als der Wirkstoff A (70 %).

## Patentansprüche (Patentansprüche für folgende(n) Vertragsstaat(en) : BE, CH, DE, FR, GB, IT, LI, NL, SE))

1. Verbindungen der Formel I 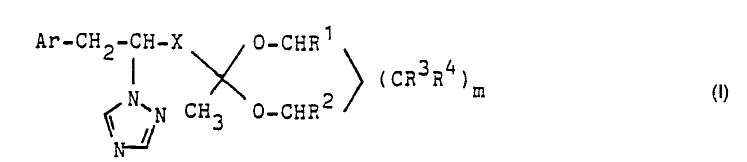 in der
R¹, R², R³, R⁴ verschieden oder gleich sind und Wasserstoff oder Alkyl mit 1 bis 5 C-Atomen bedeuten,
Ar Furanyl, Thienyl, Biphenylyl, Isoxazolyl, Naphthyl oder Phenyl bedeutet, wobei der Phenylrest durch Fluor, Chlor, Brom, lod, Nitro, Trifluormethyl, Cyano weiterhin durch Alkyl, Alkoxy oder Alkenyl mit jeweils 1 oder 2 bis 5 C-Atomen und ferner durch Phenoxy substituiert sein kann, der Isoxazolylrest durch Alkyl mit 1 bis 5 C-Atomen substituiert sein kann und
X eine -CO-, -CH(OH)-, -CR⁵(OH)- oder -CH(OR6)-Gruppe bedeutet, wobei R⁵ einen Alkylrest mit 1 bis 4 C-Atomen oder einen Alkenylrest mit 2 bis 4 C-Atomen oder einen Benzylrest, der gegebenenfalls durch Halogen substituiert sein kann, bedeutet,
R⁶ einen Alkylrest mit 1 bis 8 C-Atomen oder einen gegebenenfalls durch Chlor substituierten Alkenylrest mit 2 bis 5 C-Atomen oder einen Alkinylrest mit 3 oder 4 C-Atomen oder Benzyl bedeutet, wobei der Benzylrest durch Fluor, Chlor, Brom, Nitro, Cyano, Trifluormethyl, ferner durch Alkyl oder Alkoxy mit jeweils 1 bis 4 C-Atomen substituiert sein kann, oder R⁶ einen ―CO―R⁷-Rest bedeutet, wobei R⁷ einen gegebenenfalls durch Halogen, C₁-C₄-Alkoxy, Oxo (= 0) oder Carboxy-C₁-C₄-alkyl substituierten Alkylrest mit 1 bis 5 C-Atomen oder einen aromatischen Rest bedeutet und
m eine ganze Zahl von 0 bis 3 sein kann.

2. Verbindungen der Formel I gemäß Anspruch 1, dadurch gekennzeichnet, daß Ar Biphenylyl, Naphthyl oder Phenyl bedeutet, wobei der Phenylrest durch Fluor, Chlor, Brom, Trifluormethyl, Cyano, weiterhin durch Alkyl oder Alkoxy mit jeweils 1 bis 3 C-Atomen und ferner durch Phenoxy substituiert sein kann und X C = 0, CH(OH) oder CH(OR⁶) bedeutet.

3. Verfahren zur Herstellung von Verbindungen der Formel I gemäß Anspruch 1, dadurch gekennzeichnet, daß man
a) 1,2,4-Triazol mit einem alpha-Bromketon der Formel II 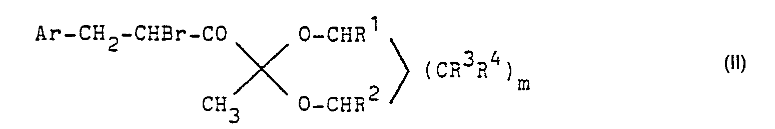 worin R¹ bis R⁴, Ar und m die oben angegebene Bedeutung haben oder
b) ein Arylmethylhalogenid der Formel III 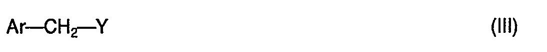 worin Ar die oben genannte Bedeutung hat und Y Chlor oder Brom ist, mit einem β-Triazolylketal der Formel IV 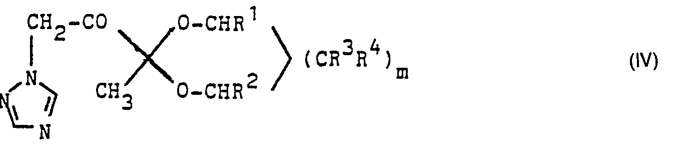 worin R¹ bis R⁴ und m die oben genannte Bedeutung haben, umsetzt, und die zo erhaltenen Verbindungen gegebenenfalls reduziert und verethert oder verestert und gegebenenfalls eine Säure oder ein Metallsalz addiert.

4. Fungizides Mittel, enthaltend eine Verbindung gemäß Anspruch 1 und einen festen oder flüssigen Trägerstoff.

5. Fungizides Mittel, enthaltend eine Verbindung gemäß Anspruch 1 und einen festen oder flüssigen Trägerstoff.

6. Verfahren zur Bekämpfung von Pilzen, dadurch gekennzeichnet, daß man eine Verbindung der Formel gemäß Anspruch 1 auf die Pilze einwirken läßt.

7. Verfahren zur Herstellung von Fungiziden, dadurch gekennzeichnet, daß man eine Verbindung der Formel mit festen oder flüssigen Trägerstoffen mischt.

8. Verfahren zur vorbeugenden Bekämpfung von Pilzen, dadurch gekennzeichnet, daß man eine Verbindung der Formel auf durch Pilzbefall bedrohte Materialien, Flächen, Pflanzen oder Saatgüter einwirken läßt.

## Patentansprüche (Patentansprüche für folgende(n) Vertragsstaat(en) : AT))

1. Fungizides Mittel, enthaltend eine Verbindung der Formel I 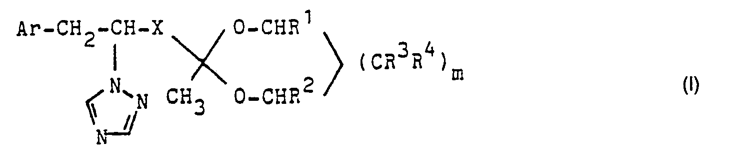 in aer
R¹, R², R³, R⁴ verschieden oder gleich sind und Wasserstoff oder Alkyl mit 1 bis 5 C-Atomen bedeuten,
Ar Furanyl, Thienyl, Biphenylyl, Isoxazolyl, Naphthyl oder Phenyl bedeutet, wobei der Phenylrest durch Fluor, Chlor, Brom, lod, Nitro, Trifluormethyl, Cyano weiterhin durch Alkyl, Alkoxy oder Alkenyl mit jeweils 1 oder 2 bis 5 C-Atomen und ferner durch Phenoxy substituiert sein kan, der Isoxazolylrest durch Alkyl mit 1 bis 5 C-Atomen substituiert sein kann und
X eine -CO-, -CH(OH)-, -CR⁵(OH)- oder ―CH(OR⁶)-Gruppe bedeutet, wobei R⁵ einen Alkylrest mit 1 bis 4 C-Atomen oder einen Alkenylrest mit 2 bis 4 C-Atomen oder einen Benzylrest, der gegebenenfalls durch Halogen substituiert sein kann, bedeutet,
R⁶ einen Alkylrest mit 1 bis 8 C-Atomen oder einen gegebenenfalls durch Chlor substituierten Alkenylrest mit 2 bis 5 C-Atomen oder einen Alkinylrest mit 3 oder 4 C-Atomen oder Benzyl bedeutet, wobei der Benzylrest durch Fluor, Chlor, Brom, Nitro, Cyano, Trifluormethyl, ferner durch Alkyl oder Alkoxy mit jeweils 1 bis 4 C-Atomen substituiert sein kann, oder R⁶ einen -CO-R⁷-Rest bedeutet, wobei R⁷ einen gegebenenfalls durch Halogen, C₁-C₄-Alkoxy, Oxo (= 0) oder Carboxy-C₁-C₄-alkyl substituierten Alkylrest mit 1 bis 5 C-Atomen oder einen aromatischen Rest bedeutet und
m eine ganze Zahl von 0 bis 3 sein kann.

2. Fungizides Mittel, enthaltend eine Verbindung gemäß Anspruch 1 und einen festen oder flüssigen Trägerstof.

3. Verfahren zur Bekämpfung von Pilzen, dadurch gekennzeichnet, daß man eine Verbindung der Formel I gemäß Anspruch 1 auf die Pilze einwirken läßt.

4. Verfahren zur Herstellung von Fungiziden, dadurch gekennzeichnet, daß man eine Verbindung der Formel I mit festen oder flüssigen Trägerstoffen mischt.

5. Verfahren zur vorbeugenden Bekämpfung von Pilzen, dadurch gekennzeichnet, daß man eine Verbindung der Formel 1 auf durch Pilzbefall bedrohte Materialien, Flächen, Pflanzen oder Saatgüter einwirken läßt.

6. Verfahren zur Herstellung von Verbindungen der Formel I gemäß Anspruch 1, dadurch gekennzeichnet, daß man
a) 1,2,4-Triazol mit einem alpha-Bromketon der Formel II 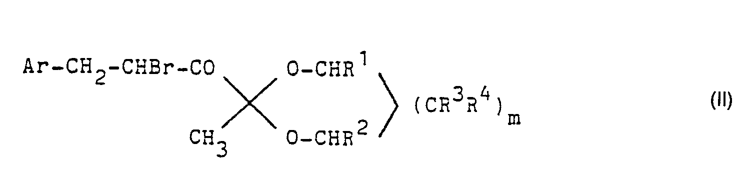 worin R¹ bis R⁴, Ar und m die oben angegebene Bedeutung haben oder
b) ein Arylmethylhalogenid der Formel III 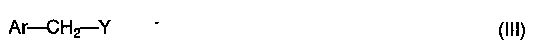 worin Ar die oben genannte Bedeutung hat und Y Chlor oder Brom ist, mit einem ß-Triazolylketal der Formel IV 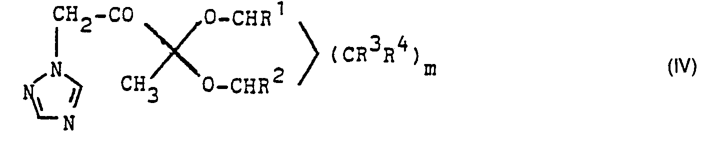 worin R¹ bis R⁴ und m die oben genannte Bedeutung haben, umsetzt, und die so erhaltenen Verbindungen gegebenenfalls eine Säure oder ein Metallsalz addiert.

7. Fungizides Mittel gemäß Anspruch 1, dadurch gekennzeichnet, daß Ar Biphenylyl, Naphthyl oder Phenyl bedeutet, wobei der Phenylrest durch Fluor, Chlor, Brom, Trifluormethyl, Cyano, weiterhin durch Alkyl oder Alkoxy mit jeweils 1 bis 3 C-Atomen und ferner durch Phenoxy substituiert sein kann und X C = 0, CH(OH) oder CH(OR⁶) bedeutet.

## Claims (Claims for the following Contracting State(s) : BE, CH, DE, FR, GB, IT, LI, NL, SE)

1. A compound of the formula I 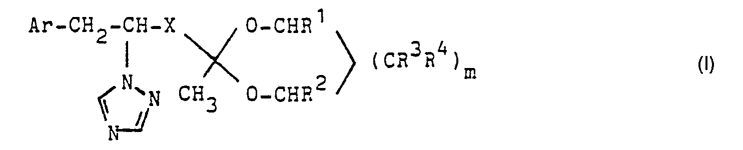 where
R¹, R², R³ und R⁴ are identical or different and each denotes hydrogen or alkyl of 1 to 5 carbon atoms,
Ar is furanyl, thienyl, biphenylyl, isoxyzolyl, naphthyl or phenyl, it being possible for phenyl to be substituted by fluorine, chlorine, bromine, iodine, nitro, trifluoromethyl or cyano, or by alkyl, alkoxy or alkenyl each having 1 or 2 to 5 carbon atoms, or by phenoxy, and for isoxazolyl to be substituted by alkyl of 1 to 5 carbon atoms,
X is a -CO-, -CH(OH)-, -CR⁵(OH)- or -CH(OR)⁶-group, where R⁵ is alkyl of 1 to 4 carbon atoms or alkenyl of 2 to 4 carbon atoms or benzyl optionally substituted by halogen,
R⁶ is alkyl of 1 to 8 carbon atoms or an optionally chlorine-substituted alkenyl of 2 to 5 carbon atoms or alkynyl of 3 or 4 carbon atoms or benzyl, it being possible for benzyl to be substituted by fluorine, chlorine, bromine, nitro, cyano or trifluoromethyl, or by alkyl or alkoxy each having 1 to 4 carbon atoms, or R⁶ is a -CO-R⁷ radical, where R⁷ is alkyl of 1 to 5 carbon atoms, optionally substituted by halogen, C₁―C₄-alkoxy, oxo (= 0) or carboxy-Cᵢ-C₄-alkyl, or is an aromatic radical, and
m is an integer from 0 to 3.

2. A compound of the formula I as claimed in claim 1, where Ar is biphenylyl, naphthyl or phenyl, it being possible for phenyl to be substituted by fluorine, chlorine, bromine, trifluoromethyl or cyano, or by alkyl or alkoxy each having 1 to 3 carbon atoms, or by phenoxy, and X is C = 0, CH(OH) or CH(OR)⁶.
3. A process for the preparation of a compound of the formula I as claimed in claim 1, wherein
a) 1,2,4-triazole is reacted with an α-bromoketone of the formula II 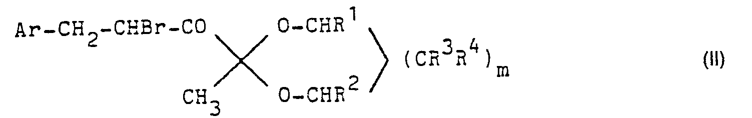 where R¹, R², R³, R⁴, Ar and m have the above meanings, or
b) an aryl methyl halide of the formula III 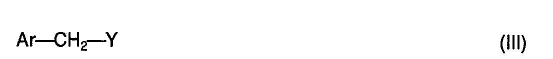 where Ar has the above meanings, and Y is chlorine or bromine, is reacted with a ß-triazolyl ketal of the formula IV 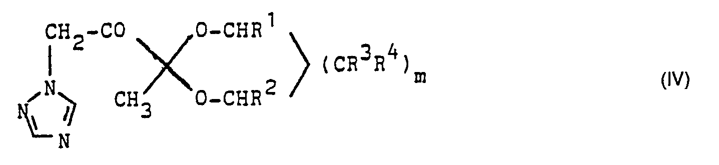 where R¹, R², R³, R⁴ and m have the above meanings.

4. A fungicidal agent containing a compound as claimed in claim 1.

5. A fungicidal agent containing a compound as claimed in claim 1 and a solid or liquid carrier.

6. A process for combating fungi, wherein a compound of the formula I as claimed in claim 1 is allowed to act on the fungi.

7. A process for producing a fungicide, wherein a compound of the formula I is mixed with a solid or liquid carrier.

8. A process for the prophylactic control of fungi, wherein a compound of the formula I is allowed to act on the materials, areas, plants or seed threatened by fungal attack.

## Claims (Claims for the following Contracting State(s) : AT)

1. A fungicidal agent containing a compound of the formula I 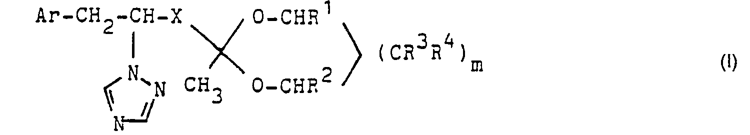 where
R¹, R², R³ and R⁴ are identical or different and each denotes hydrogen or alkyl of 1 to 5 carbon atoms,
Ar is furanyl, thienyl, biphenylyl, isoxazolyl, naphthyl or phenyl, it being possible for phenyl to be substituted by fluorine, chlorine, bromine, iodine, nitro, trifluoromethyl or cyano, or by alkyl, alkoxy or alkenyl each having 1 or 2 to 5 carbon atoms, or by phenoxy, and for isoxazolyl to be substituted by alkyl of 1 to 5 carbon atoms,
X is a -CO-, -CH(OH)-, -CR⁵(OH)- or -CH(OR⁶)-group, where R⁵ is alkyl of 1 to 4 carbon atoms or alkenyl of 2 to 4 carbon atoms or benzyl optionally substituted by halogen,
R⁶ is alkyl of 1 to 8 carbon atoms or an optionally chlorine-substituted alkenyl of 2 to 5 carbon atoms or alkynyl of 3 or 4 carbon atoms or benzyl, it being possible for benzyl to be substituted by fluorine, chlorine, bromine, nitro, cyano or trifluoromethyl, or by alkyl or alkoxy each having 1 to 4 carbon atoms, or R⁶ is a ―CO―R⁷ radical, where R⁷ is alkyl of 1 to 5 carbon atoms optionally substituted by halogen, C₁-C₄-alkoxy, oxo (= 0) or carboxy-Cᵢ-C₄-alkyl, or is an aromatic radical, and
m is an integer from 0 to 3.

2. A fungicidal agent containing a compound as claimed in claim 1 and a solid or liquid carriers.

3. A process for combating fungi, wherein a compound of the formula I as claimed in claim 1 is allowed to act on the fungi.

4. A process for producing a fungicide, wherein a compound of the formula I is mixed with a solid or liquid carrier.

5. A process for the prophylactic control of fungi, wherein a compound of the formula I is allowed to act on the materials, areas, plants or seed threatened by fungal attack.

6. A process for the preparation of a compound of the formula I as claimed in claim 1, wherein
a) 1,2,4-triazole is reacted with an a-bromoketone of the formula II 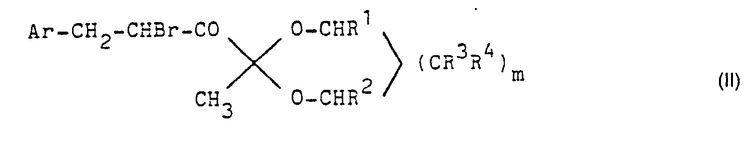 where R¹, R², R³, R⁴, Ar and m have the above meanings, or
b) an aryl methyl halide of the formula III 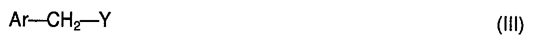 where Ar has the above meanings, and Y is chlorine or bromine, is reacted with a β-triazolyl ketal of the formula IV

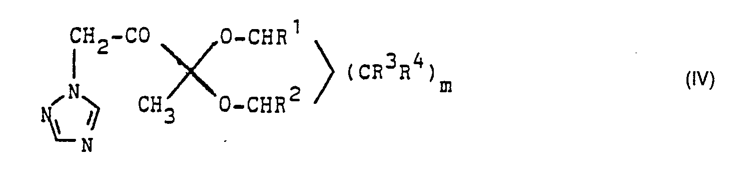

7. A fungicidal agent as claimed in claim 1, where Ar is biphenylyl, naphthyl or phenyl, it being possible for phenyl to be substituted by fluorine, chlorine, bromine, trifluoromethyl or cyano, or by alkyl or alkoxy each having 1 to 3 carbon atoms, or by phenoxy, and X is C = 0, CH(OH) or CH(OR⁶).

## Revendications (Revendications pour l'(les) Etat(s) contractant(s) suivant(s) : BE, CH, DE, FR, GB, IT, LI, NL, SE)

1. Composés de la formule I 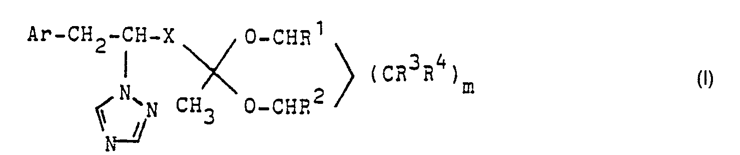 dans laquelle
R¹, R², R³ et R⁴, qui peuvent être identiques ou différents, désignent chacun un atome d'hydrogène ou un radical alkyle en C₁ à C₅,
Ar représente un groupe furannyle, thiényle, biphénylyle, isoxazolyle, naphtyle ou phényle, le groupe phényle pouvant être substitué par du fluor, du chlore, du brome ou de l'iode, des groupes nitro, trifluorométhyle ou cyano, des radicaux alkyle, alcoxy ou alcényle en Ci ou C₂ à C₅ ou par un groupe phénoxy et le groupe isoxazolyle pouvant être substitué par des radicaux alkyle en C₁ à C₅,
X désigne un groupe -CO-, -CH(OH)-, ―CR⁵(OH)― ou -CH(OR⁶)-, où R⁵ est un radical alkyle en C₁ à C₄ ou un radical alcényle en C₂ à C₄ ou un groupe benzyle éventuellement halo-substitué et
R⁶ un radical alkyle en Ci à C₈ ou un radical alcényle en C₂ à C₅ éventuellement chloro-substitué ou un radical alcynyle en C₃ ou C₄ ou un groupe benzyle, ce dernier pouvant être substitué par du fluor, du chlore ou du brome, des groupes nitro, cyano ou trifluorométhyle ou des radicaux alkyle ou alcoxy en C₁ à C₄, ou R⁶ est un groupe ―CO―R⁷, où R⁷ désigne un radical alkyle en C₁ à C₅ éventuellement halo-, alcoxy (en Ci à C₄)-, oxo (= 0)- ou carboxy-alkyl (en C₁ à C₄)-substitué ou un groupe aromatique et
m est un nombre entier valant 0 à 3.

2. Composés de la formule I selon la revendication 1, caractérisés en ce que Ar désigne un groupe biphénylyle, naphtyle ou phényle, le groupe phényle pouvant être substitué par du fluor, du chlore ou du brome, des groupes trifluorométhyle ou cyano, des radicaux alkyle ou alcoxy en C₁ à C₃ ou un groupe phénoxy et X est un groupe -C = 0, -CH(OH) ou -CH(OR⁶).

3. Procédé de préparation de composés de la formule I selon la revendication 1, caractérisé en ce que l'on fait réagir
a) du 1,2,4-triazole avec une alpha-bromo-cétone de la formule Il 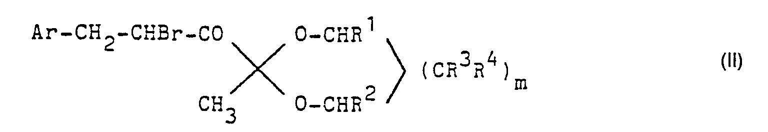 dans laquelle R¹ à R⁴, Ar et m possèdent la signification indiquée plus haut, ou
b) un halogénure d'aryl-méthyle de la formule III 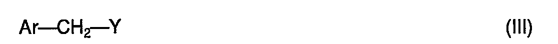 dans laquelle Ar possède la signification définie et Y désigne un atome de chlore ou de brome, avec un bêta-triazolyl-cétal de la formule IV 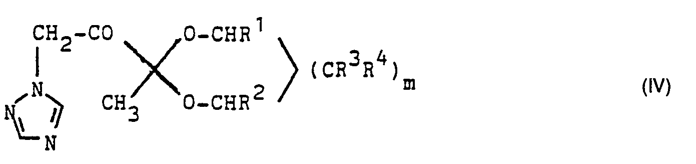 dans laquelle R¹ à R⁴ et m possèdent la signification définie plus haut, les composés obtenus pouvant le cas échéant être soumis à une réduction, une éthérification ou une estérification ou additionnés d'un acide ou d'un sel de métal.

4. Composition fongicide, contenant un composé selon la revendication 1.

5. Composition fongicide, contenant un composé selon la revendication 1 et une matière support solide ou liquide.

6. Procédé de lutte contre les champignons, caractérisé en ce que l'on fait agir sur les champignons un composé de la formule I selon la revendication 1.

7. Procédé de préparation de compositions fongicides, caractérisé en ce que l'on mélange un composé de la formule I à des matières supports solides ou liquides.

8. Procédé de lutte préventive contre les champignons, caractérisé en ce que l'on fait agir sur les matériaux, surfaces, plantes ou semences risquant d'être attaqués par des champignons un composé de la formule I.

## Revendications (Revendications pour l'(les) Etat(s) contractant(s) suivant(s) : AT)

1. Composition fongicide, contenant un composé de la formule I 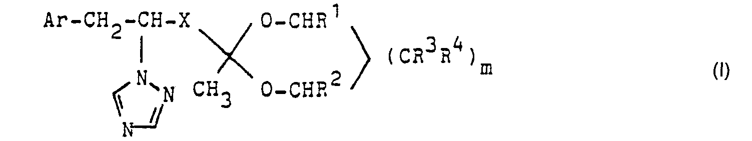 dans laquelle
R¹, R², R³ et R⁴, qui peuvent être identiques ou différentes, désignent chacun un atome d'hydrogène ou un radical alkyle en C₁ à C₅,
Ar représente un groupe furannyle, thiényle, biphénylyle, isoxazolyle, naphtyle ou phényle, le groupe phényle pouvant être substitué par du fluor, du chlore, du brome ou de l'iode, des groupes nitro, trifluorométhyle ou cyano, des radicaux alkyle, alcoxy ou alcényle en Ci ou C₂ à C₅ ou par un groupe phénoxy et le groupe isoxazolyle pouvant être substitué par des radicaux alkyle en C₁ à C₅,
X désigne un groupe -CO-, -CH(OH)-, -CR⁵(OH)- ou ―CH(OR⁶)―, où R⁵ est un radical alkyle en C₁ à C₄ ou un radical alcényle en C₂ à C₄ ou un groupe benzyle éventuellement halo-substitué et
R⁶ un radical alkyle en C₁ à C₈ ou un radical alcényle en C₂ à C₅ éventuellement chloro-substitué ou un radical alcynyle en C₃ ou C₄ ou un groupe benzyle, ce dernier pouvant être substitué par du fluor, du chlore ou du brome, des groupes nitro, cyano ou trifluorométhyle ou des radicaux alkyle ou alcoxy en C₁ à C₄, ou R⁶ est un groupe ―CO―R⁷, où R⁷ désigne un radical alkyle en C₁ à C₅ éventuellement halo-, alcoxy (en C₁ à C₄)- oxo (= 0)- ou carboxy-alkyl (en C₁ à C₄-substitué ou un groupe aromatique et
m est un nombre entier valant 0 à 3.

2. Composition fongicide, contenant un composé selon la revendication 1 et une matière support solide ou liquide.

3. Procédé de lutte contre les champignons, caractérisé en ce que l'on fait agir sur les champignons un composé de la formule I selon la revendication 1.

4. Procédé de préparation de compositions fongicides, caractérisé en ce que l'on mélange un composé de la formule I à des matières supports solides ou liquides.

5. Procédé de lutte préventive contre les champignons, caractérisé en ce que l'on fait agir sur les matériaux, surfaces, plantes ou semences risquant d'être attaqués par des champignons un composé de la formule I.

6. Procédé de préparation de composés de la formule I selon la revendication 1, caractérisé en ce que l'on fait réagir
a) du 1,2,4-triazole avec une alpha-bromo-cétone de la formule Il 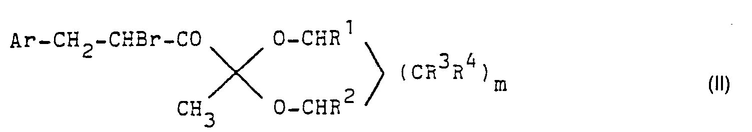 dans laquelle R¹ à R⁴, Ar et m possèdent la signification indiquée plus haut, ou
b) un halogénure d'aryl-méthyle de la formule III 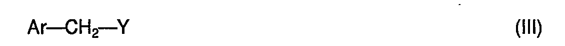 dans laquelle Ar possède la signification définie et Y désigne un atome de chlore ou de brome, avec un bêta-triazolyl-cétal de la formule IV 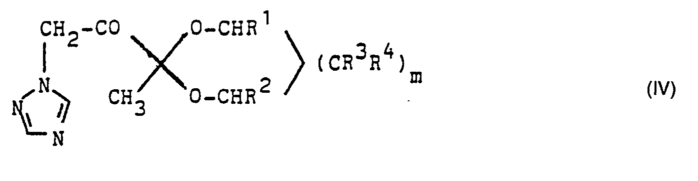 dans laquelle R¹ à R⁴ et m possèdent la signification définie plus haut, les composés obtenus pouvant le cas échéant être soumis à une réduction, une éthérification ou une estérification ou additionnés d'un acide ou d'un sel d'un métal.

7. Composition fongicide selon la revendication 1, caractérisée en ce que Ar désigne un groupe biphénylyle, naphtyle ou phényle, le groupe phényle pouvant être substitué par du fluor, du chlore ou du brome, des groupes trifluorométhyle ou cyano, des radicaux alkyle ou alcoxy en C₁ à C₃ ou un groupe phénoxy et X est un groupe -C = 0, -CH(OH) ou -CH(OR⁶).
